(19) Europäisches Patentamt
European Patent Office
Office européen des brevets

(11) Veröffentlichungsnummer: **0 044 974**
**B1**

# (12) EUROPÄISCHE PATENTSCHRIFT

(45) Veröffentlichungstag der Patentschrift: **16.05.84**

(51) Int. Cl.[3]: **C 07 C 69/712**, C 07 C 67/307

(21) Anmeldenummer: **81105207.5**

(22) Anmeldetag: **04.07.81**

(54) **Verfahren zur Herstellung von 2-Aryloxy-2-halogenpropionsäureverbindungen.**

(30) Priorität: **29.07.80 DE 3028625**

(43) Veröffentlichungstag der Anmeldung: **03.02.82 Patentblatt 82/5**

(45) Bekanntmachung des Hinweises auf die Patenterteilung: **16.05.84 Patentblatt 84/20**

(84) Benannte Vertragsstaaten: **BE CH DE FR GB LI**

(56) Entgegenhaltungen: **DE - A - 2 720 654**

(73) Patentinhaber: **BASF Aktiengesellschaft, Carl-Bosch-Strasse 38, D-6700 Ludwigshafen (DE)**

(72) Erfinder: **Husslein, Gerd, Dr. Chem., Herrenbergstrasse 2, D-6702 Bad Duerkheim (DE)**
Erfinder: **Hamprecht, Gerhard, Dr. Chem., Rote-Turm-Strasse 28, D-6940 Weinheim (DE)**

EP 0 044 974 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 2-Aryloxy-2-halogenpropionsäureverbindungen

Die Erfindung betrifft ein Verfahren zur Herstellung von 2-Aryloxy-2-halogenpropionsäureverbindungen durch Umsetzung von 2-Aryloxypropionsäureverbindungen mit N-Halogencarbonsäureimiden in Gegnwart von aliphatischen Halogenkohlenwasserstoffen, aliphatischen und/oder cycloaliphatischen Kohlenwasserstoffen als Lösungsmittel, von 0,0001 bis 0,001 Mol Chlor oder Brom, bezogen auf Ausgangsstoff III, und von 0,005 bis 0,05 Gewichtsprozent Wasser, bezogen auf die Gewichtsmenge Ausgangsstoff III, und 0,0005 bis 0,005 Mol Azo-bis-isobutyronitril und/oder Dibenzoylperoxid, bezogen auf Ausgangsstoff III.

Es ist aus der deutschen Offenlegungsschrift 27 20 654 bekannt, daß man Alkancarbonsäurederivate, z. B. 4-Chlorphenoxyessigsäure-tert.-butylester mit N-Bromsuccinimid in Gegenwart eines Lösungsmittels und in Gegenwart von Radikalbildnern, wie Diebenzoylperoxid oder Azo-bis-isobuttersäurenitril zu Brom-(4-chlorphenoxy)-essigsäure-tert.-butylester umsetzen kann. Es wird lediglich im Falle des 4-Chlorphenoxyessigsäure-tert.-butylesters angegeben, daß man bei dieser Umsetzung 2 Stunden Reaktionszeit benötigt und eine Ausbeute von 76 Prozent erhält. Führt man das Verfahren mit entsprechenden Propionsäureestern und insbesondere im großtechnischen Maßstab durch, so benötigt man wesentlich längere Reaktionszeiten, z. B. 8 bis 15 Stunden, um Ausbeuten von 60 bis 80% der Theorie zu erzielen.

Aber auch im Falle von Essigsäurederivaten ist mit unbefriedigenden Reaktionszeiten und Ausbeuten zu rechnen. Die Acta Chimica Academiae Scientiarum Hungaricae, Tomus 79 (1973), Seiten 419 bis 432, zeigt, daß aus 4-Chlorphenoxyessigsäureäthylester mit N-Bromsuccinimid in kochendem Tetrachlorkohlenstoff trotz Dibenzoylperoxid als Katalysator, einer Reaktionszeit von 17 Stunden und hoher Aktivierung durch Belichtung nur eine Ausbeute von 52,8 Prozent des Brom-Derivats erhalten wird.

Es wurde nun gefunden, daß man 2-Aryloxy-2-halogenpropionsäureverbindungen der Formel

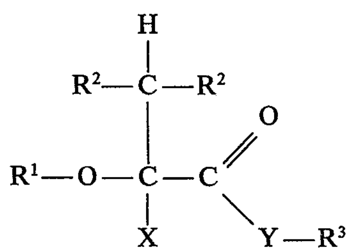

(I)

worin Y ein Sauerstoffatom oder den Rest

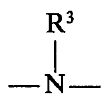

bedeutet, $R^1$ einen aromatischen Rest bezeichnet, die einzelne Reste $R^2$ und $R^3$ gleich oder verschieden sein können und jeweils für ein Wasserstoffatom, einen Alkylrest mit 1 bis 18 Kohlenstoffatomen oder einen Cycloalkylrest mit 5 bis 8 Kohlenstoffatomen stehen, $R^3$ darüber hinaus auch einen araliphatischen oder aromatischen Rest bedeutet, und X ein Chloratom oder Bromatom bezeichnet, durch Umsetzung von 2-Aryloxypropionsäureverbindungen in Gegenwart von N-Halogencarbonsäureimiden und gewünschtenfalls Halogenkohlenwasserstoffen als Lösungsmitteln vorteilhaft erhält, wenn man 2-Aryloxypropionsäureverbindungen der Formel

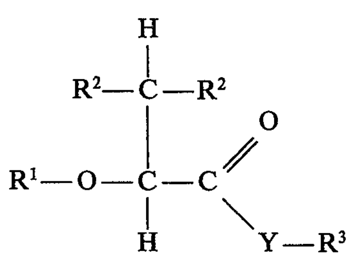

(II)

worin $R^1$, $R^2$, $R^3$ und Y die vorgenannten Bedeutungen besitzen, mit N-Halogencarbonsäureimiden der Formel

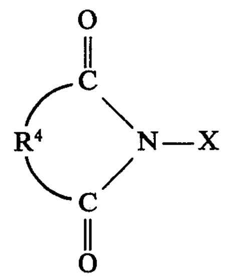

(III)

worin X die vorgenannte Bedeutung besitzt und $R^4$ für einen Alkylenrest mit 1 bis 6 Kohlenstoffatomen steht, in Gegenwart von aliphatischen Halogenkohlenwasserstoffen, aliphatischen und/oder cycloaliphatischen Kohlenwasserstoffen als Lösungsmittel, von 0,0001 bis 0,001 Mol Chlor oder Brom, bezogen auf ein Mol Ausgangsstoff III, und von 0,005 bis 0,05 Gewichtsprozent Wasser, bezogen auf die Gewichtsmenge Ausgangsstoff III, und 0,0005 bis 0,005 Mol Azo-bis-isobutyronitril und/oder Dibenzoylperoxid, bezogen auf Ausgangsstoff III, umsetzt.

Verwendet man 2-(2,4-Dichlorphenoxy)-propionsäuremethylester und N-Bromsuccinimid als Ausgangsstoffe, kann der Reaktionsablauf des erfindungsgemäßen Verfahrens durch folgendes Formelschema wiedergegeben werden:

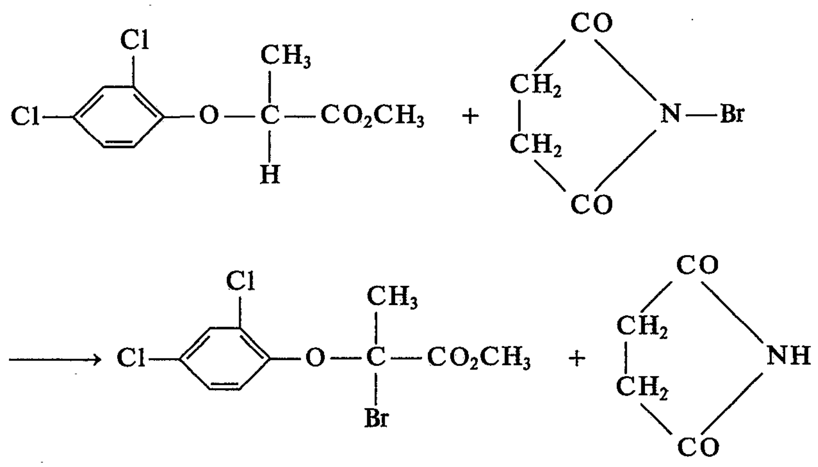

Im Vergleich zu den bekannten Verfahren liefert das Verfahren nach der Erfindung auf einfacherem und wirtschaftlicherem Wege 2-Aryloxy-2-halogenpropionsäureverbindungen in besserer Ausbeute, Raum-Zeit-Ausbeute und Reinheit. Die Reaktionszeit bei diesen Propionsäurederivaten ist erheblich kürzer. Alle diese vorteilhaften Ergebnisse sind überraschend, denn man hätte in Hinblick auf insbesondere die ungarische Veröffentlichung eine wesentlich langwierige Umsetzung, da ja auch die Kohlenwasserstoffzahl der erfindungsgemäßen Alkancarbonsäuren größer ist, und entsprechend niedrige Ausbeute erwarten müssen. Angesichts der Anwesenheit von Wasser und der Reaktivität der Komponenten hätte man daneben Nebenreaktionen annehmen müssen, über die die vorgenannten Veröffentlichungen, die unter Ausschluß von Wasser arbeiten, keine Aussage machen. Man hätte so als Ergebnis des erfindungsgemäßen Verfahrens schwer trennbare heterogene Reaktionsgemische mit entsprechend verringerter Ausbeute und Reinheit des Endstoffs erwartet.

Bevorzugte Ausgangsstoffe II und dementsprechend bevorzugte Endstoffe I sind solche, in deren Formeln Y ein Sauerstoffatom oder den Rest

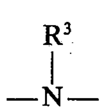

bedeutet, $R^1$ einen Phenylrest, Naphthylrest oder einen durch 1, 2, oder 3 Chloratome, Bromatome, Nitrogruppen, Cyanogruppen und/oder perhalogenierte Alkyl- oder Alkoxygruppen mit jeweils 1 bis 6 Kohlenstoffatomen substituierten Phenylrest oder einen unsubstituierten oder in vorgenannter Weise substituierten Diphenylrest bezeichnet, die einzelnen Reste $R^2$ und $R^3$ gleich oder verschieden sein können und jeweils für ein Wasserstoffatom, einen Alkylrest mit 1 bis 8 Kohlenstoffatomen, oder einen Cycloalkylrest mit 5 bis 8 Kohlenstoffatomen stehen, $R^3$ darüber hinaus auch einen Aralkylrest oder Alkylarylrest mit 7 bis 12 Kohlenstofftomen, einen Phenylrest oder einen durch 1, 2 oder 3 Chloratome, Bromatome, Nitrogruppen, Cyanogruppen und/oder perhalogenierte Alkyl- oder Alkoxygruppen mit jeweils 1 bis 6 Kohlenstoffatomen substituierten Phenylrest stehen, bedeutet. Die vorgenannten Reste

können noch durch unter den Reaktionsbedingungen inerte Atome oder Gruppen, z. B. Alkylgruppen oder Alkoxygruppen mit jeweils 1 bis 4 Kohlenstoffatomen, Arylreste substituierende Chloratome und/oder Bromatome, substituiert sein.

So kommen folgende Ausgangsstoffe II in Betracht: 2-Phenoxypropionsäure und in 3-Stellung einfach oder gleich oder unterschiedlich zweifach durch Methyl-, Ethyl-, n-Propyl-, iso-Butyl-, sek.-Butyl-, tert.-Butyl-, n-Pentyl-, 1-Methylbutyl-, 1-Ethylpropyl-, 1-Ethyl-2-methyl-propyl-, 1'-Chlorethyl-, Trichlormethyl-, 2'-Chlorethyl-, 1'-Chlorpropyl-, 2'-Chlorpropyl-, 1'-Chlor-2'-propyl-, 3'-Brombutyl-, Methoxyethyl-, Methoxyisopropyl-, Ethoxy-tert.-butyl-, Methoxy-tert.-butyl-, Cyclopentyl-, Cyclohexyl-, 2-Chlorcyclohexylgruppen substituierte 2-Phenoxypropionsäuren; unsubstituierte oder in vorgenannter Weise substituierte Methyl-, Ethyl-, Propyl-, Isopropyl-, Butyl-, Isobutyl-, sek.-Butyl-, tert.-Butyl-, Cyclohexyl-, Cyclopentyl-, Phenyl-, Benzyl-ester vorgenannter Säuren unsubstituierte oder in vorgenannter Weise substituierte, einfach oder zweifach gleich oder unterschiedlich durch Methyl-, Ethyl-, Propyl-, iso-Propyl-, Butyl-, iso-Butyl-, sek.-Butyl-, tert.-Butyl-, Cyclohexyl-, Phenyl-, Benzyl-, N-substituierte Propionsäureamide vorgenannter Säuren; unsubstituierte oder in vorgenannter Weise substituierte, in 2-, 3-, 4-, 5-, 6-Stellung am Phenylkern einfach oder gleich oder verschieden 2fach oder 3fach durch Chloratome, Bromatome, Methyl-, Ethyl-, Propyl-, iso-Propyl-, Butyl-, iso-Butyl-, sek.-Butyl-, tert.-Butyl-, Methoxy-, Ethoxy-, Propoxy-, iso-Propoxy-, Butoxy-, iso-Butoxy-, sek.-Butoxy-, tert.-Butoxy-, Nitrogruppen substituierte 2-Phenoxypropionsäuren-, -propionsäureester- und -propionsäureamide.

Besonders bevorzugte Ausgangsstoffe II sind:

2-(2,4-Dichlorphenoxy)-propionsäuremethylester, 2-(4-Chlorphenoxy)-propionsäureethylester,
2-(4-Chlorphenoxy)-propionsäure-methylester, 2-(4-Chlorphenoxy)-buttersäureethylester,
2-(2-Brom-4-chlorphenoxy)-propionsäuremethylester,
2-(2,4-Dichlorphenoxy)-propionsäureisooctylester,
2-(2,4,5-Trichlorphenoxy)-propionsäure-tert.-butylester,
2-(4-Phenylphenoxy)-propionsäuremethylester, 2-(4-Nitro-phenoxy)-propionsäureethylester,
2-(2-Dichlorphenoxy)-iso-valeriansäureethylester,
2-(2,4-Dichlorphenoxy)-propionsäure-N,N-dimethylamid.

Die Umsetzung wird bei einer Temperatur von — 10 bis +150° C, vorzugsweise von 20 bis 120° C, insbesondere von 40 bis 120° C, drucklos oder unter Druck, kontinuierlich oder diskontinuierlich durchgeführt. Man verwendet als unter den Reaktionsbedingungen inerte Lösungsmittel aliphatische Halogenkohlenwasserstoffe, insbesondere Chlorkohlenwasserstoffe, z. B. Tetrachloräthylen, 1,1,2,2- oder 1,1,1,2-Tetrachloräthan, Amylchlorid, Dichlorpropan, Methylenchlorid, Dichlorbutan, Isopropylbromid, Chloroform, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid; bevorzugt Tetrachlorkohlenstoff und Tetrachloräthylen; aliphatische oder cycloaliphatische Kohlenwasserstoffe, z. B. Heptan, Cyclohexan, Methylcyclohexan, Dekalin, Petroläther, Hexan, Ligroin; und entsprechende Gemische. Zweckmäßig verwendet man das Lösungsmittel in einer Menge von 100 bis 10 000 Gewichtsprozent, vorzugsweise von 500 bis 2 000 Gewichtsprozent, bezogen auf Ausgangsstoff II.

Die Umsetzung wird in Gegenwart von Chlor und insbesondere Brom, in einer Menge von 0,0001 bis 0,001, vorzugsweise von 0,0002 bis 0,0006 Mol Halogen, bezogen auf ein Mol Ausgangsstoff III, durchgeführt. Anmeldungsgemäße N-Halogencarbonsäureimide der Formel III sind solche, in deren Formeln X ein Chloratom und insbesondere ein Bromatom bedeutet und $R^4$ für einen Alkylenrest mit 2 bis 6, insbesondere 2 bis 4 Kohlenstoffatomen steht. Die vorgenannten Reste können noch durch unter den Reaktionsbedingungen inerte Atome oder Gruppen, z. B. Alkylgruppen oder Alkoxygruppen mit jeweils 1 bis 4 Kohlenstoffatomen, substituiert sein. Es kommen z. B. als Ausgangsstoffe III in Frage: Die N-Chlorimide und vorzugsweise die N-Bromimide der Bernsteinsäure, Glutarsäure, Adipinsäure, Pimelinsäure, Korksäure, Azelainsäure, Sebacinsäure; bevorzugt sind N-Bromsuccinimid und N-Chlorsuccinimid. Man kann die Ausgangsstoffe III in stöchiometrischer Menge oder im Überschuß, vorteilhaft in einer Menge von 0,9 bis 2, vorzugsweise von 1 bis 1,5 Mol Ausgangsstoff III je Mol Ausgangsstoff II, verwenden.

Wasser wird in einer Menge von 0,005 bis 0,05, insbesondere 0,005 bis 0,01 Gewichtsprozent Wasser, bezogen auf die Gewichtsmenge Ausgangsstoff III, verwendet. Man kann Wasser den Ausgangskomponenten getrennt zufügen; zweckmäßig gibt man es in Gestalt eines Gemisches mit Halogen und einem Anteil an dem verwendeten Halogenkohlenwasserstoff zu. Vorteilhaft verwendet man 0,01- bis 3-, insbesondere 0,5- bis 1gewichtsprozentige Lösungen von Chlor oder 0,1- bis 3-, insbesondere 0,5- bis 1gewichtsprozentige Lösungen von Brom im jeweiligen Halogenkohlenwasserstoff und setzt ihnen das Wasser bis zur Sättigung zu. Man verwendet die Radikalbildner Azo-bis-isobutyronitril oder Dibenzoylperoxid zweckmäßig in einer Menge von 0,001 bis 0,005 Mol Radikalbildner je Mol Ausgangsstoff III.

Die Reaktion kann wie folgt durchgeführt werden: Ein Gemisch von Ausgangsstoff II und III, zusammen mit Halogen, Wasser, Lösungsmittel und Radikalbildner wird während 0,5 bis 5 Stunden bei

der Reaktionstemperatur gehalten. Aus dem Reaktionsgemisch wird der Endstoff I in üblicher Weise, z. B. durch fraktionierte Kristallisation, abgetrennt.

Die nach dem Verfahren der Erfindung herstellbaren Endstoffe I sind wertvolle Ausgangsstoffe für die Herstellung von Pharmazeutika, Pflanzenschutzmitteln und Farbstoffen. Bezüglich der Verwendung wird auf die vorgenannten Veröffentlichungen verwiesen.

Die in den folgenden Beispielen aufgeführten Teile bedeuten Gewichtsteile.

Beispiel 1

Eine gesättigte, wäßrige Lösung aus 0,01 Teilen Brom und einem Teil Tetrachlorkohlenstoff wird zu einem Gemisch von 37,5 Teilen 2-(2,4-Dichlorphenoxy)-propionsäuremethylester, 26,7 Teilen N-Bromsuccinimid, 0,1 Teilen Azo-bis-isobutyronitril und 300 Teilen Tetrachlorkohlenstoff zugegeben. Man setzt nach 45 Minuten 0,1 Teile Azo-bis-isobuttersäurenitril und 0,01 Teile Brom in 1 Teil Tetrachlorkohlenstoff bei 70° C zu und rührt bei 77° C weitere 45 Minuten nach. Man kühlt auf 40° C ab, filtriert und engt das Filtrat im Vakuum ein. Man erhält 45 Teile (91,5% der Theorie) 2-Brom-2-(2,4-Dichlorphenoxy)-propionsäuremethylester vom $n_D^{22} = 1,5390$.

Beispiel 2 (Vergleich)

37,5 Teile 2-(2,4-Dichlorphenoxy)-propionsäuremethylester und 26,7 Teile N-Bromsuccinimid werden in 300 Teilen Tetrachlorkohlenstoff zum Sieden erhitzt, wobei jeweils alle 45 Minuten 0,1 Teile Azo-bis-isobuttersäurenitril hinzugegeben werden. Man erhitzt so lange unter Rückfluß, bis das entstehende Succinimid auf dem Lösungsmittel schwimmt. Insgesamt benötigt man 0,8 Teile Azo-bis-isobuttersäurenitril und eine Gesamtreaktionszeit von 10 Stunden. Danach wird auf 0° C abgekühlt, filtriert und das Filtrat im Vakuum eingeengt. Man erhält 36,9 Teile (82% der Theorie) 2-Brom-2-(2,4-Dichlorphenoxy)-propionsäuremehylester vom $n_D^{22} = 1,5395$.

**Patentanspruch**

Verfahren zur Herstellung von 2-Aryloxy-2-halogenpropionsäureverbindungen der Formel

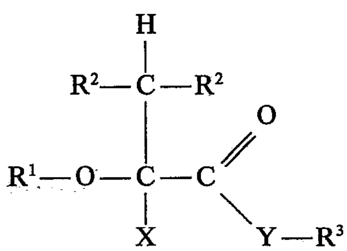

(I)

worin Y ein Sauerstoffatom oder den Rest

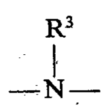

bedeutet, $R^1$ einen aromatischen Rest bezeichnet, die einzelnen Reste $R^2$ und $R^3$ gleich oder verschieden sein können und jeweils für ein Wasserstoffatom, einen Alkylrest mit 1 bis 18 Kohlenstoffatomen oder einen Cycloalkylrest mit 5 bis 8 Kohlenstoffatomen stehen, $R^3$ darüber hinaus auch einen araliphatischen oder aromatischen Rest bedeutet, und X ein Chloratom oder Bromatom bezeichnet, durch Umsetzung von 2-Aryloxypropionsäureverbindungen in Gegenwart von N-Halogencarbonsäureimiden und gewünschtenfalls Halogenkohlenwasserstoffen als Lösungsmitteln, dadurch gekennzeichnet, daß man 2-Aryloxypropionsäureverbindungen der Formel

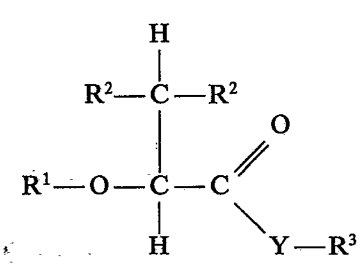

(II)

worin $R^1$, $R^2$, $R^3$ und Y die sogenannten Bedeutungen besitzen, mit N-Halogencarbonsäureimiden der Formel

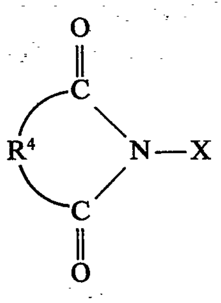

(III)

worin X die vorgenannte Bedeutung besitzt und $R^4$ für einen Alkylenrest mit 1 bis 6 Kohlenstoffatomen steht, in Gegenwart von aliphatischen Halogenkohlenwasserstoffen, aliphatischen und/oder cycloaliphatischen Kohlenwasserstoffen als Lösungsmitteln, von 0,0001 bis 0,001 Mol Chlor oder Brom, bezogen auf ein Mol Ausgangsstoff III, und von 0,005 bis 0,05 Gewichtsprozent Wasser, bezogen auf die Gewichtsmenge Ausgangsstoff III, und 0,0005 bis 0,005 Mol Azo-bis-isobutyronitril und/oder Dibenzoylperoxid, bezogen auf Ausgangsstoff III, umsetzt.

**Claim**

A process for the preparation of 2-aryloxy-2-halopropionic acid compounds of the formula

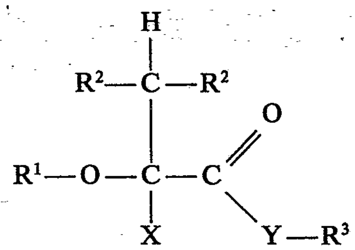

(I)

where Y is oxygen or

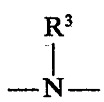

$R^1$ is an aromatic radical, the $R^2$'s and $R^3$'s can be identical or different and each is hydrogen, alkyl of 1 to 18 carbon atoms or cycloalkyl of 5 to 8 carbon atoms, and $R^3$ can also be an araliphatic or aromatic radical, and X is chlorine or bromine, by reacting a 2-aryloxypropionic acid compound with an N-halocarboxylic acid imide in the presence or absence of a halohydrocarbon as solvent, wherein a 2-aryloxypropionic acid compound of the formula

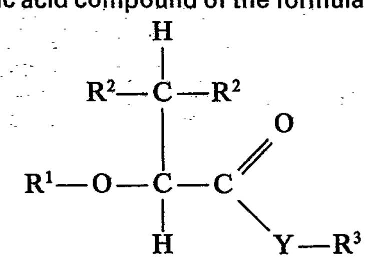

(II)

where $R^1$, $R^2$, $R^3$ and Y have the above meanings, is reacted with an N-halocarboxylic acid imide of the formula

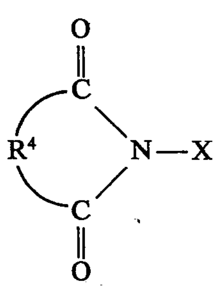

(III)

where X has the above meanings and $R^4$ is alkylene of 1 to 6 carbon atoms, in the presence of an aliphatic halohydrocarbon, an aliphatic hydrocarbon, a cycloaliphatic hydrocarbon or a mixture thereof as solvent, and of from 0.0001 to 0.001 mole of chlorine or bromine per mole of starting material III, from 0.005 to 0.05 per cent by weight of water, based on the amount of starting material III, and from 0.0005 to 0.005 mole of azo-bis-isobutyronitrile and/or dibenzoyl peroxide per mole of starting material III.

**Revendication**

Procédé de préparation de dérivés d'acides aryloxy-2 halo-2 propioniques de la formule

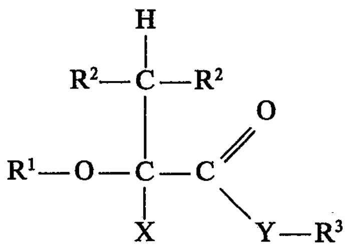

(I)

dans laquelle Y désigne un atome d'oxygène ou un groupe

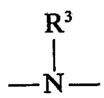

$R^1$ un reste aromatique et $R^2$ et $R^3$, qui peuvent être identiques ou différents, représentent chacun un atome d'hydrogène, un radical alcoyle en $C_1$ à $C_{18}$ ou un groupe cycloalcoyle en $C_5$ à $C_8$, $R^3$ pouvant en outre être un reste araliphatique ou aromatique, et X désigne un atome de chlore ou de brome, par réaction de dérivés d'acides aryloxy-2 propioniques et d'imides d'acides N-halo-carboxyliques, éventuellement en présence d'hydrocarbures halogénés comme solvants, caractérisé en ce que l'on fait réagir un dérivé d'acide aryloxy-2 propionique de la formule

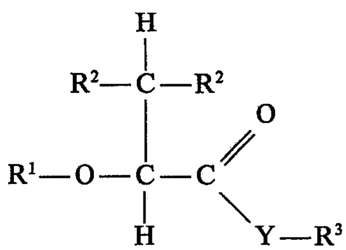

(II)

dans laquelle $R^1$, $R^2$, $R^3$ et Y possèdent les significations définies, avec un imide d'acide N-halo-carboxylique de la formule

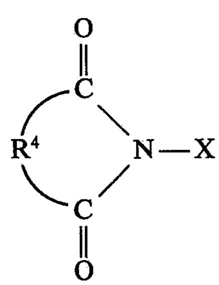

(III)

dans laquelle X possède la signification définie et $R^4$ représente un groupe alcoylène en $C_1$ à $C_6$, en présence d'hydrocarbures aliphatiques halogénés ou d'hydrocarbures aliphatiques et(ou) cycloaliphatiques comme solvants, de 0,0001 à 0,001 mole de chlore ou de brome par mole de composé de départ III, de 0,005 à 0,05% en poids par rapport au composé de départ III d'eau et de 0,0005 à 0,005 mole par rapport au composé de départ III d'azo-bis-isobutyro-nitrile et(ou) de peroxyde de dibenzoyle.